# EUROPÄISCHE PATENTSCHRIFT

(11) **EP 3 218 016 B1**
(45) Veröffentlichungstag und Bekanntmachung des Hinweises auf die Patenterteilung: **17.01.2024**
(21) Anmeldenummer: 15798318.0
(22) Anmeldetag: 14.10.2015
(51) Int. Cl.: A61L 2/10, C02F 1/32

(54) **VORRICHTUNG ZUR BEHANDLUNG EINES FLUIDS MIT UV-STRAHLUNG**
DEVICE FOR TREATING A FLUID WITH UV RADIATION
DISPOSITIF DE TRAITEMENT D'UN FLUIDE PAR RAYONNEMENT UV

(30) Priorität: 15.10.2014 DE 102014015049
(43) Veröffentlichungstag der Anmeldung: 20.09.2017
(73) Patentinhaber: EurA AG, 73479 Ellwangen (DE); Friedrich-Schiller-Universität Jena, 07743 Jena (DE)
(72) Erfinder: SCHMIDT, Dirk, 98529 Suhl (DE); WONDRACZEK, Lothar, 07743 Jena (DE)
(74) Vertreter: Weihrauch, Frank
(86) Internationale Anmeldenummer: PCT/DE2015/000507
(87) Internationale Veröffentlichungsnummer: WO 2016/058580

(56) Entgegenhaltungen:
- WO-A2-99/27970
- US-A1- 2010 224 562
- US-A1- 2014 110 351

## Beschreibung

Die Erfindung betrifft eine Vorrichtung zur Behandlung eines Fluids, mittels UV-Strahlung, insbesondere zur Desinfektion und Entkeimung, wobei es sich bei dem zu behandelnden Fluid vorliegend insbesondere um Wasser zur Trinkwasserbereitstellung handelt.

Vorrichtungen zur Wasserentkeimung sind aus dem Stand der Technik bereits bekannt. Hierbei wird in der Regel das zu entkeimende Wasser in einen entsprechenden Behälter eingeleitet oder durch einen Behälter durchgeleitet und in diesem einer Bestrahlung mit UV-Strahlung, insbesondere UV-C-Strahlung, unterzogen.

Die UV-Strahlung wird hierzu mittels UV-Lampen, insbesondere mittels UV-Röhrenlampen bereitgestellt.

Diese sind häufig in dem Wandungsbereich des Behälters so angeordnet, dass die UV-Strahlung von dort aus in das zu entkeimende Wasser eingeleitet wird. Eine derartige Anordnung der UV-Lampen wird auch als positive Bestrahlungsgeometrie bezeichnet.

Der Nachteil derartiger Vorrichtungen besteht insbesondere darin, dass es problematisch ist Geometrien auszubilden, bei denen die Strahlungsintensität in Richtung der Behältermittenachse nicht übermäßig abnimmt um eine ausreichend intensive Bestrahlung des zu entkeimenden Wassers in allen Bereichen zu gewährleisten.

Alternativ sind aus dem Stand der Technik Vorrichtungen bekannt, welche eine negative Bestrahlungsgeometrie aufweisen, was bedeutet, dass die UV-Lampe, beispielsweise in einem Tauchrohr, im Inneren des Behälters angeordnet ist und somit von innen heraus die UV-Strahlung in das zu entkeimende Wasser eingebracht wird.

Eine solche Vorrichtung wird beispielsweise in der Druckschrift DE 10 2008 021 301 A1 als UV-Reaktor beschrieben. Der UV-Reaktor weist in diesem Fall ein Gehäuse sowie einen Hohlkörper und eine in dem Hohlkörper angeordnete Lichtquelle zur Bestrahlung eines Mediums mit UV-Licht auf, wobei das zu bestrahlende Medium durch einen Hohlraum zwischen dem Hohlkörper und dem Gehäuse hindurchströmt.

Derartige Vorrichtungen weisen insbesondere den Nachteil auf, dass die UV-Lampe gegenüber dem zu entkeimenden Wasser flüssigkeitsdicht abgeschirmt werden muss. Hierbei muss insbesondere die Strahlungsdurchlässigkeit der verwendeten Abschirmung berücksichtigt werden, sodass für diesen Zweck nur auf wenige und mitunter teure Materialien zurückgegriffen werden kann. Ferner besteht auch hier ein Abfall der Strahlungsintensität in Abhängigkeit von der Entfernung zur UV-Lampe,

Allen bekannten Vorrichtungen ist zudem der Nachteil gemein, dass die Anlagengeometrie stets an die geometrische Ausbildung der UV-Lampen angepasst werden muss, um eine ausreichend effektive Entkeimung gewährleisten zu können. Durch die Mindestbaugrößen, die röhrenförmige Bauform und die Abstrahlungscharakteristik der konventionellen UV-Lampen sind damit nachteilige Grenzen in Bezug auf die Anlagengeometrie gesetzt. Dies führt unter Umständen zu überdimensionierten und teuren Anlagengeometrien sowie zu strömungsmechanisch nachteiligen Lösungen und im Ergebnis zu einer geringen Anlageneffizienz.

Einen anderen Lösungsansatz bilden UV-LEDs, welche den Vorteil geringer Baugröße aufweisen. Dem steht jedoch der Nachteil gegenüber, dass zumindest nach gegenwärtigem Entwicklungsstand UV-LEDs sehr teuer sind und dass ferner die UV-Strahlungsleistung für eine praktische Anwendung noch zu gering ist.

Im Bereich der UV-LEDs offenbart die Druckschrift DE 10 2010 047 318 A1 einen weiteren Lösungsansatz zur Bereitstellung einer Bestrahlungseinrichtung, bei welcher ein zu bestrahlendes Medium durch eine Bestrahlungskammer geleitet wird. Außerhalb der Bestrahlungskammer ist eine UV-Strahlungsquelle angeordnet, welche unter anderem als UV-LED ausgebildet sein kann und deren emittierte UV-Strahlung in die Bestrahlungskammer eingeleitet und in dieser mehrfach reflektiert wird.

Der Nachteil einer solchen Lösung besteht insbesondere darin, dass zum einen besonders leistungsstarke UV-LEDs benötigt werden sowie dass die Intensität der UV-Strahlung, je nach Dichte des zu bestrahlenden Mediums, mit zunehmendem Reflektionsweg unterschiedlich abnimmt. Ferner ist es technisch aufwendig, den erforderlichen hohen Reflexionsgrad bereitszustellen und auch bei Verschmutzung der Bestrahlungskammer aufrecht zu erhalten. Beispiele mit strangförmigem UV-Element finden sich in US6468433, US 2010/224562 und US 2014/110351.

Die Aufgabe der Erfindung bildet es, unter Überwindung der Nachteile des Standes der Technik eine Vorrichtung zur Behandlung eines Fluids mit UV-Strahlung bereitzustellen, welche eine hohe Bestrahlungseffizienz und eine hohe geometrische Variabilität aufweist, bei welcher der Bestrahlungsbereich besonders klein ausgebildet sein kann und welche zudem kostengünstig bereitstellbar ist.

Die Aufgabe wird durch eine Vorrichtung mit den im Anspruch 1 aufgeführten Merkmalen gelöst. Bevorzugte Weiterbildungen ergeben sich aus den Unteransprüchen.

Als Fluid im Sinne der erfindungsgemäßen Vorrichtung zur Behandlung eines Fluids sind Flüssigkeiten wie insbesondere Wasser, wobei es sich auch um kontaminiertes oder verunreinigtes Wasser handeln kann, und wässrige Lösungen zu verstehen, ohne hierauf beschränkt zu sein.

Eine erfindungsgemäße Vorrichtung zur Behandlung eines Fluids mit UV-Strahlung weist ein Behältnis auf, in welchem das zu behandelnde Fluid aufnehmbar ist. Das Fluid kann hierbei für die Dauer der Behandlung zeitweise stationär in dem Behältnis aufgenommen werden oder dieses während der Behandlung durchströmen, wobei das Behältnis je nach Anwendungsfall insbesondere als Rohr oder als einseitig beziehungsweise beidseitig geschlossenes Gefäß ausgebildet sein kann. Das Behältnis muss jedoch nicht notwendig geschlossen sein. Als Behältnis im Sinne der erfindungsgemäßen Vorrichtung ist vielmehr jedes Mittel anzusehen, dass die räumliche Anordnung des Fluids beeinflusst. So kann es sich bei dem Behältnis beispielsweise auch um eine Anordnung von Platten handeln.

Des Weiteren weist die erfindungsgemäße Vorrichtung ein UV-Leiterelement auf, welches strangförmig ausgebildet ist und aus einem Material mit einer besonders guten Leitfähigkeit für UV-Strahlung besteht. Das UV-Leiterelement ist vorzugsweise flexibel ausgebildet, sodass dieses vorteilhaft an unterschiedliche, geometrische Formen des Behältnisses angepasst werden kann.

Das UV-Leiterelement weist vorliegend einen Einleitungsabschnitt auf, in welchem die UV-Strahlung in das UV-Leiterelement einleitbar ist. Der Einleitungsabschnitt kann hierbei beispielsweise durch eine Stirnseite des UV-Leiterelements gebildet werden.

Ferner weist das UV-Leiterelement einen Abstrahlabschnitt auf, mit welchem eine laterale Abstrahlung der UV-Strahlung in das zu behandelnde Wasser bereitstellbar ist.

Der Abstrahlabschnitt ist erfindungsgemäß innerhalb des Behältnisses und somit so in Bezug zu dem zu behandelnden Fluids angeordnet, sodass das zu behandelnde Fluid mit UV-Strahlung beaufschlagbar ist. Vorzugsweise ist eine negative Bestrahlungsgeometrie bereitstellbar, welche eine besonders effektive Bestrahlung des zu behandelnden Fluids ermöglicht.

Das UV-Leiterelement wird erfindungsgemäß durch ein UV-leitfähiges Material mit einer optischen Transparenz, vorzugsweise in einem Spektralbereich von 200 bis 500 nm, gebildet. Es weist zudem vorzugsweise einen Brechungsindex von mindestens 1,4 auf.

Der Spektralbereich von 200 bis 500 nm bietet hierbei den Vorzug, dass durch das UV-Leiterelement alle Wellenlängen der UV-Strahlung, also in den Bereichen der UV-A, UV-B und UV-C Strahlung, abgestrahlt werden können. Je nach Anwendungsfall kann das Materiel des UV-Leiterelements auch andere optische Transparenzen in eingeschränkten Spektralbereichen aufweisen, sodass beispielsweise nur bestimmte Wellenlängen der UV-Strahlung abgestrahlt werden.

Die laterale Ausleitung eines UV-Lichtstromes über die Mantelfläche ist durch eine geeignete Modifikation des Materials des UV-Leiterelements, vorzugsweise durch Strukturierung der Mantelfläche, insbesondere Aufrauen, Beschichten oder Anätzen erreichbar. Ferner kann die Modifikation zur Erzielung der Ausleitung des UV-Lichtstromes insbesondere auch durch gezielte Einschlüsse im Inneren des Materials des UV-Leiterelements oder durch andere Mittel erreicht werden. Bei den Einschlüssen kann es sich insbesondere um Lufteinschlüsse handeln.

Je nach Anwendungsfall ist die Strukturierung der Mantelfläche besonders bevorzugt im linearen Verlauf des UV-Leiterelements in der Weise zunehmend, dass im linearen Verlauf ein zunehmender Anteil des UV-Lichtstromes ausgeleitet wird. Dem liegt zu Grunde, dass der UV-Lichtstrom im Inneren des UV-Leiterelements aufgrund der ausgeleiteten Anteile im linearen Verlauf stets abnimmt. Durch einen, im linaren Verlauf steigenden, Ausleitungsanteil kann somit bei geeigneter Auslegung ein etwa gleicher UV-Teillichtstrom pro Längeneinheit erreicht werden, wodurch ein besonders gleichmäiger UV-Strahlungseintrag in das zu behandelnde Fluid erreicht werden kann. Je nach Anwendungsfall ist es vorliegend auch möglich, innerhalb des Abstrahlabschnittes sogenannte Hot Spots vorzusehen, in welchen eine besonders starke Abstrahlung der UV-Strahlung bereitstellbar ist. Damit ist eine einfache Anpassung an bestimmte Geometrien des Behältnisses möglich.

Als weiteres Element weist die Vorrichtung zur Behandlung von Fluid mit UV-Strahlung erfindungsgemäß eine UV-Strahlungsquelle auf, mit welcher die UV-Strahlung zur Einleitung in das UV-Leiterelement bereitstellbar ist.

Je nach Anwendungsfall ist die UV-Strahlungsquelle vorzugsweise dem UV-Leiterelement derart zugeordnet, dass die UV-Strahlung unmittelbar in den Einleitungsabschnitt einbringbar ist. Beispielsweise kann die UV-Strahlungsquelle hierbei im Bereich einer Stirnseite des UV-Leiterelements angeordnet sein oder im Bereich der Stirnseite an dem UV-Leiterelement anliegen.

Für besondere Anwendungsfälle ist es auch möglich, UV-Leiterelement und UV-Strahlungsquelle räumlich getrennt voneinander innerhalb der Vorrichtung anzuordnen, sodass nur ein definierter Teil der UV-Strahlung in das UV-Leiterelement eingeleitet wird.

Erfindungsgemäß können als UV-Strahlungsquelle sämtliche UV-Strahlungsquellen wie beispielsweise Xe-Entladungslampen angewendet werden. Es können vorliegend jedoch auch UV-Strahlung emittierende LEDs zum Einsatz kommen, welche gegenüber anderen UV-Strahlungsquellen besonders klein ausgebildet sein können.

Gegenüber herkömmlichen Vorrichtungen zur Behandlung von Fluid mit UV-Strahlung weist die vorliegende Lösung insbesondere den Vorteile auf, dass die räumliche Anordnung der UV-Strahlungsquelle von der räumlichen Anordnung des zu behandelnden Fluids entkoppelt wird und somit die Geometrie des Bestrahlungsbereichs in dem Behältnis unabhängig von der Geometrie der UV-Stahlungsquelle optimiert gestaltet werden kann. Es besteht eine große geometrische Variabilität. Einen weiteren besonderen Vorteil bildet es, dass durch die laterale Abstrahlung eine gleichmäßige Bestrahlungsleistung über eine größere Länge möglich ist. Ferner kann bei einem flexibel ausgebildeten UV-Leiterelement auch bei gekrümmten oder in anderer Weise besonderen Geometrien des Bestrahlungsbereichs, wie beispielsweise Abschattungsbereichen, eine angepasste Bestrahlungsleistung erreicht werden. Überraschend wurde mit der technischen Lösung ein Weg gefunden, bei dem auch ein Fluid mit einem Brechungsindex von größer 1,2 mit den genannten Vorteilen mit UV-Strahlung beaufschlagt werden kann. Insgesamt besteht der Vorteil, dass eine besonders hohe Effizienz der UV-Bestrahlung erreicht werden kann.

Die Erfindung sieht vor, dass das UV-Leiterelement eine Mehrzahl Fasern aufweist, wobei die Fasern vorzugsweise aus einem flexiblen Fasermaterial bestehen und separat oder innerhalb eines oder mehrerer Faserbündel vorliegen können und wobei jede Faser einen Einleitungssowie einen Abstrahlabschnitt aufweist.

Die Fasern weisen bevorzugt einen Durchmesser von kleiner oder gleich 0,5 mm, besonders bevorzugt von kleiner oder gleich 0,25 mm auf. Dies bietet den besonderen Vorteil einer möglichen Miniaturisierung des Bereichs, in dem die UV-Strahlung in das Fluid eingebracht wird.

Ein weiterer besonderer Vorteil der Fasern besteht vorliegend in deren besonders hoher Flexibilität, welche die Realisierung unterschiedlichster, je nach Anwendungsfall auch komlizierter Geometrien des UV-Leiterelements ermöglicht. In diesem Zusammenhang kann beispielsweise ein spiralförmiges UV-Leiterelement mit einer optimierten Bestrahlungseffizienz bereitgestellt werden. Vorzugsweise folgt die Form des UV-Leiterelements durch desse Flexibilität dabei passiv der Form einer unterstützenden Struktur, beispielsweise einer Rohrwendel. Hierbei kann die unterstützende Struktur auch durch das Behältnis selbst oder durch Teile hiervon gebildet werden.

Zudem wurde als weiterer Vorteil der hier aufgezeigten Variante gefunden, dass sich die Fasern des UV-Leiterelements stets in Strömungsrichtung eines strömenden Mediums ausrichten. Auf diese Weise können bei einer Ausführungsvariante der Vorrichtung, bei welcher das Behältnis von dem zu behandelnden Fluid durchströmt wird, stets eine Ausrichtung des UV-Leiterelements, vorzugsweise einer Vielzahl von Fasern, in dem Behältnis im Wesentlichen parallel zu der Strömungsrichtung und somit eine kontinuierlich gleichmäßige Bestrahlung des Fluids in dem Behältnis gewährleistet werden. Ferner muss als weiterer Vorteil das strömungsmechanische Design des Behältnisses praktisch nicht in Abhängigkeit von der Geometrie der UV-Strahlungsquelle angepasst werden.

Ferner sind in einer bevorzugten Weiterbildung der Vorrichtung die UV-Strahlungsquelle und der Einleitungsabschnitt außerhalb des Behältnisses angeordnet.

Hierbei ist beispielsweise eine Anordnung des UV-Leiterelements und der UV-Strahlungsquelle möglich, bei welcher das UV-Leiterelement eine Wandung des Behältnisses durchsetzt, sodass der gesamte Abstrahlabschnitt im Inneren des Behältnisses angeordnet ist.

Alternativ ist es vorliegend auch möglich, dass das UV-Leiterelement derart in das Behältnis eingehangen wird, dass der Abstrahlabschnitt vollständig in das zu behandelnde Fluid eintaucht.

Ein technologischer Vorteile der hier aufgezeigten Weiterbildung besteht insbesondere darin, dass das Behältnis nicht gemäß einem benötigten Bauraum für die UV-Strahlungsquelle gestaltet sein muss, so dass auch Behältnisse mit sehr geringen Abmessungen, wie beispielsweise sehr dünne Röhrchen mit Durchmessern im Millimeterbereich, möglich sind
Ein weiterer Vorteil der Weiterbildung besteht darin, dass die UV-Strahlungsquelle nicht zwingend gegenüber dem zu behandelnden Fluid abgedichtet werden muss. Auf diese Weise können die Aufwendungen für eine Abdichtung der UV-Strahlungsquelle gegenüber dem zu behandelnden Fluid vermieden und somit die Gesamtbereitstellungskosten für die erfindungsgemäße Vorrichtung niedrig gehalten werden. Zudem werden die Betriebssicherheit erhöht und ein besonderer einfacher Austausch der UV-Strahlungsquelle begünstigt.

Darüber hinaus besteht ein weiterer Vorteil der obenstehenden Weiterbildung darin, dass lediglich der Abstrahlabschnitt des UV-Leiterelements innerhalb des Behältnisses angeordnet werden muss und somit geringstmögliche Abmessungen des Behältnisses bereitgestellt werden können.

In einer bevorzugten Ausbildung der Vorrichtung besteht das UV-Leiterelement aus Silikatglas und insbesondere aus Kieselglas.

Neben den optimalen optischen Eigenschaften, wie einer optischen Transparenz im Spektralbereich von 200 bis 500 nm sowie einem Brechungsindex von 1,4, weist Silikatglas im vorliegenden Fall den besonderen Vorteil auf, dass es eine besonders gute Stabilität gegenüber dem zu behandelnden Fluid besitzt.

Die Erfindung sieht vor, dass die Behandlung des Fluids in einem Durchströmungsverfahren erfolgt und dass die Vorrichtung ein Durchströmungselement aufweist, welches innerhalb des Behältnisses angeordnet ist.

Das Durchströmungselement ist vorzugsweise so innerhalb des Behältnisses angeordnet, dass die Außenwandungen des Durchströmungselements an den Innenwandungen des Behältnisses anliegen, so dass der im Wesentlichen gesamte wirksame Durchströmungsquerschnitt auf das Durchströmungselement entfällt.

Das Durchströmungselement weist vorliegend Durchströmungsausnehmungen auf, deren Geometrie und Anzahl nach strömungsmechanischen Gesichtspunkten sowie der gewählten Anzahl der Fasern des UV-Leiterelements gewählt werden können und welche erfindungsgemäß eine Doppelfunktion aufweisen. Zum einen dienen die Durchströmungsausnehmungen zur Aufnahme und beabstandeten Positionierung der Fasern, wobei vorzugsweise in jeder Durchströmungsausnehmung mindestens eine Faser angeordnet ist. Zum anderen dienen die Durchströmungsöffnungen dazu, dass das zu behandelnde Fluid durch diese hindurchströmt und somit erstens den direkten UV-Beaufschlagungsbereich der einzelnen Fasern durchläuft und zweitens im Bereich der Durchströmungsausnehmungen eine Vergleichmäßigung der Strömung erzielt wird. Auf diese Weise kann die Bestrahlungseffizienz der Vorrichtung zusätzlich optimiert werden.

Je nach Anwendungsfall und vorhandenem Bauraum kann das Durchströmungselement unterschiedlich ausgebildet sein. So ist es vorliegend beispielsweise möglich, das Durchströmungselement als Lochblech, als Anordnung mehrerer, hintereinanderliegender Lochbleche oder nebeneinander angeordneter Rohrelemente auszubilden.

Gemäß einer vorteilhaften Weiterbildung der Erfindung weisen die Durchströmungsöffnungen eine längliche Erstreckung auf, wobei als längliche Erstreckung verstanden wird, dass die Länge mindestend das Doppelte des Druchmessers bei einer rohrförmigen Geometrie der Durchströmungsöffnung oder das Doppelte des größten lichten Maßes bei anderen Querschnitten der Durchströmungsöffnungen aufweist. Vorzugsweise wird das Durchströmungselement als langezogener Körper ausgebildet, welcher von den Durchströmungsausnehmungen entlang seiner Längsachse durchsetzt ist. Die Fasern des UV-Leiterelements sind in den Durchströmungsöffnungen im Wesentlichen parallel zu deren Längsachse positioniert. Der Querschnitt der Durchströmungsöffnungen legt damit den maximalen Abstand des zu behandelden Fluids zu einer Faser fest. Vorzugsweise werden die Länge der Durchströmungsöffnungen mindestens so groß wie die Länge des Abstrahlabschnitts und eine Anordnung des gesamten Abstrahlabschnitts in den Durchströmungsöffnungen gewählt, um eine besonders hohe Bestahlungseffizienz zu erreichen.

In einer besonders bevorzugten Variante wird das Durchflusselement aus einem UV-leitfähigem Material gebildet. Der Anteil der UV-Absorbtion durch die Wandungen der Durchströmungsöffnungen wird in diesem Fall als Vorteil reduziert. Zudem werden die UV-Beaufschlagung des Fluids zusätzlich vergleichmäßigt und die Bestrahlungseffektivität weiter erhöht.

Die Erfindung wird in Ausführungsbeispielen anhand von
- Fig. 1: schematische Darstellung einer Ausführungsform zur Behandlung von stehendem Fluid
- Fig. 2: schematische Darstellung Durchströmungslösung mit mehreren Fasern
- Fig. 3: schematische Darstellung Durchströmungslösung mit einzelner Faser und schmalem Behältnisquerschnitt
- Fig. 4: schematische Darstellung Durchströmungslösung mit Durchströmungselement
näher erläutert.

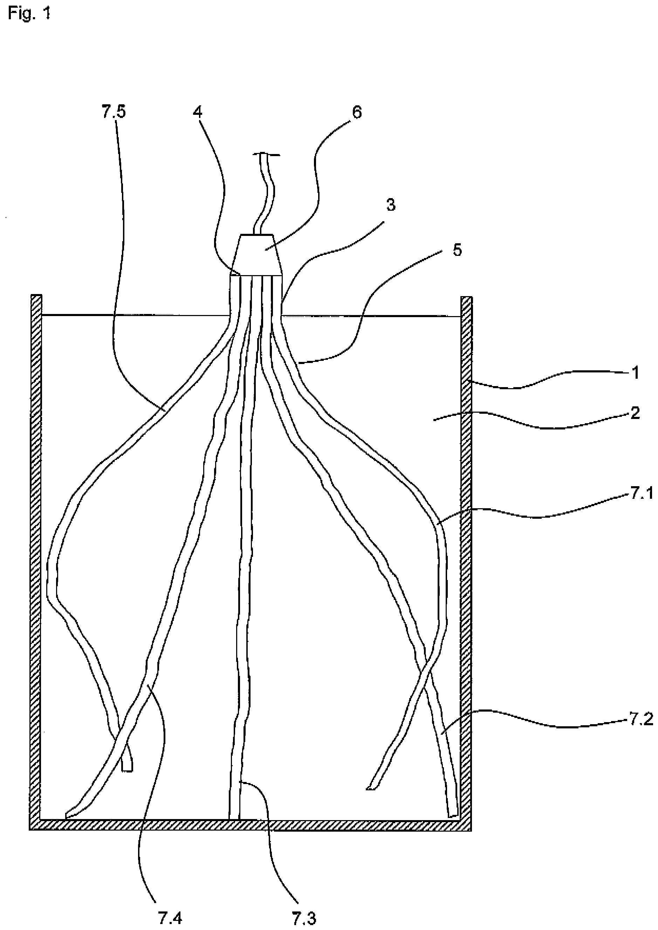

In Fig. 1 ist ein Ausführungsbeispiel der erfindungsgemäßen Lösung dargestellt, bei welcher das zu behandelnde Fluid 2 für die Dauer der Behandlung ruht. Bei dem zu behandelnden Fluid handelt es sich vorliegend um Wasser, insbesondere für die Aufbereitung zu Trinkwasser.

Im hier dargestellten Ausführungsbeispiel weist die erfindungsgemäße Vorrichtung zur Behandlung eines Fluids mit UV-Strahlung ein Behältnis 1 auf, in welchem das Fluid 2 während der Behandlung aufgenommen ist.

Des Weiteren weist die Vorrichtung ein strangförmiges UV-Leiterelement 3, welches vorliegend aus einer Mehrzahl separater und insbesondere flexibler Fasern 7 aus Kieselglas gebildet wird, sowie eine UV-Strahlungsquelle 6 zur Bereitstellung der UV-Strahlung auf. Das UV-Leiterelement 3 verfügt erfindungsgemäß über einen Einleitungsabschnitt 4, in welchem die UV-Strahlung in das UV-Leiterelement 3 eingeleitet wird, sowie über einen Abstrahlabschnitt 5, in welchem eine laterale Abstrahlung der UV-Strahlung in das Fluid 2 erfolgt.

Das UV-Leiterelement 3 und die UV-Strahlungsquelle 6 sind erfindungsgemäß derart gegenüber dem Behältnis 1 angeordnet, dass die UV-Strahlungsquelle 6 und der Einleitungsabschnitt 4 außerhalb des Behältnisses 1 und der Abstrahlabschnitt 5 innerhalb des Behältnisses 1 und insbesondere in dem Fluid 2 angeordnet sind.

Wie in Fig. 1 ebenfalls dargestellt, sind die UV-Strahlungsquelle 6 und das UV-Leiterelement 3 vorzugsweise unmittelbar aneinandergrenzend angeordnet, wobei der Einleitungsabschnitt 4 im Wesentlichen durch die Grenzfläche zwischen der UV-Strahlungsquelle 6 und dem UV-Leiterelement 3 gebildet wird.

Die Vorrichtung zeichnet sich dadurch aus, dass die Oberfläche des UV-Leiterelements 3, vorliegend der Fasern 7, in dem Abstrahlabschnitt 5 derart strukturiert ist, dass die Abstrahlung der UV-Strahlung in das Fluid 2 lateral, also seitlich beziehungsweise quer zur Längsrichtung der Fasern 7, erfolgt.

Die Fasern 7 weisen in den dargestellten Ausführungsbeispielen eine optische Transparenz im Spektralbereich von 200 bis 500 nm sowie einen Brechungsindex von mindestens 1,4 auf.

Wie aus Fig. 1 ersichtlich, weist die erfindungsgemäße Vorrichtung bei einer Behandlung von stehendem Fluid 2 in einem Behältnis 1 den besonderen Vorteil auf, dass sich die Fasern 7 aufgrund deren Flexibilität in dem Behältnis 1 und somit in dem zu behandelnden Fluid 2 ausbreiten können. Im Gegensatz zu den sonst üblichen Lösungen mit starren UV-Lampen, können auf diese Weise ein deutlich größerer Bereich des Fluids 2 durch das UV-Leiterelement 3 wirksam bestrahlt und somit eine deutlich verbesserte Bestrahlungseffizienz durch die Vorrichtung bereitgestellt werden.

Zudem besteht vorliegend die Möglichkeit, die Vorrichtung und insbesondere das UV-Leiterelement 3 während der Behandlung des Fluids 2 einer Eigenbewegung zu unterziehen und so die Fasern 7 durch das Fluid 2 zu führen.

Ein weiterer technologischer Vorteil der Vorrichtung besteht, wie aus den Figuren 1 bis 3 ersichtlich, darin, dass insbesondere die UV-Strahlungsquelle 6 stets außerhalb des Behältnisses 1 angeordnet ist und sich somit außerhalb der Einwirkung des Fluids 2 befindet. Aufgrund dieser Anordnung kann auf eine sonst übliche Abdichtung der jeweiligen UV-Strahlungsquelle gegen ein Eindringen des Fluids verzichtet werden.

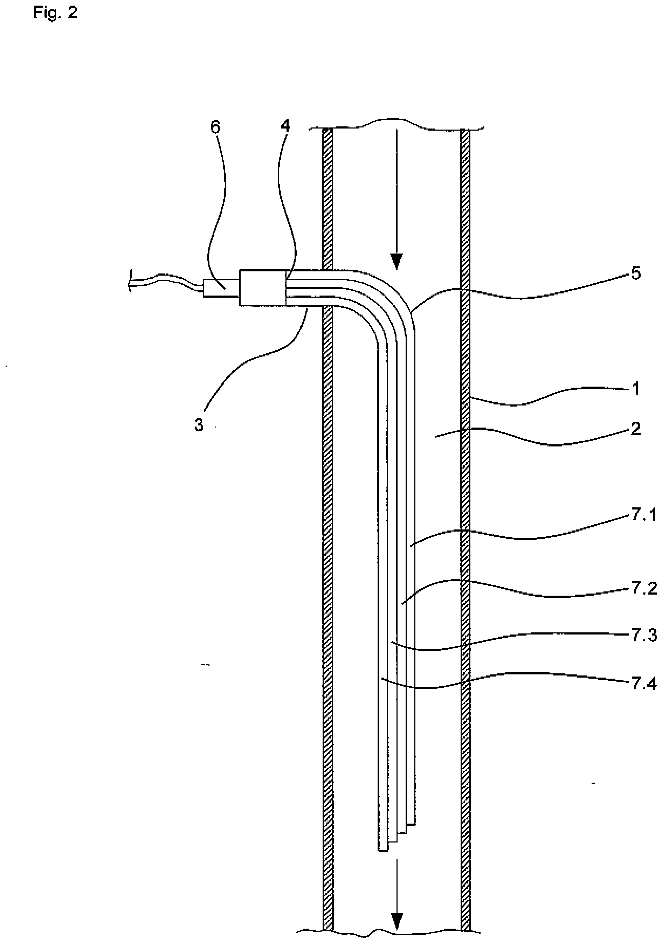

Fig. 2 zeigt ein Ausführungsbeispiel der erfindungsgemäßen Vorrichtung, bei welcher die Behandlung des Fluids 2 in einem Durchströmverfahren erfolgt. Hierzu ist das Behältnis 1 vorliegend als Rohr- beziehungsweise als Rohrabschnitt ausgebildet, durch welches das Fluid 2 strömt und welches beispielsweise in einem Fluidkreislauf eines Wohnhauses vorgesehen werden kann.

Das UV-Leiterelement 3 ist im hier dargestellten Ausführungsbeispiel so angeordnet, dass dieses das Behältnis 1 im Bereich einer Wandung derart durchsetzt, dass der Abstrahlabschnitt 5 im Inneren des Behältnisses 1 und die UV-Strahlungsquelle 6 sowie der Einleitungsabschnitt 4 außerhalb des Behältnisses 1 angeordnet sind.

Wie in Fig. 1 ist das UV-Leiterelement 3 in dem hier dargestellten Ausführungsbeispiel strangförmig ausgebildet und weist eine Mehrzahl seperate und flexible Fasern 7 auf. Das UV-Leiterelement 3 ist zudem druckdicht mit dem Behältnis 1 verbunden, wobei die Verbindung lösbar ausgebildet ist, sodass ein Entnehmen des UV-Leiterelements 3 aus dem Behältnis 1 ermöglicht wird.

Durch die Flexibilität der Fasern 7 wird es auf besonders vorteilhafte Weise ermöglicht, dass sich diese während der Behandlung des Fluids 2 entlang der Strömung ausrichten und somit ein möglichst großer Wirkungsbereich der abgestrahlten UV-Strahlung bereitgestellt wird, in welchem die Fasern 7 von dem Fluid 2 umströmt werden.

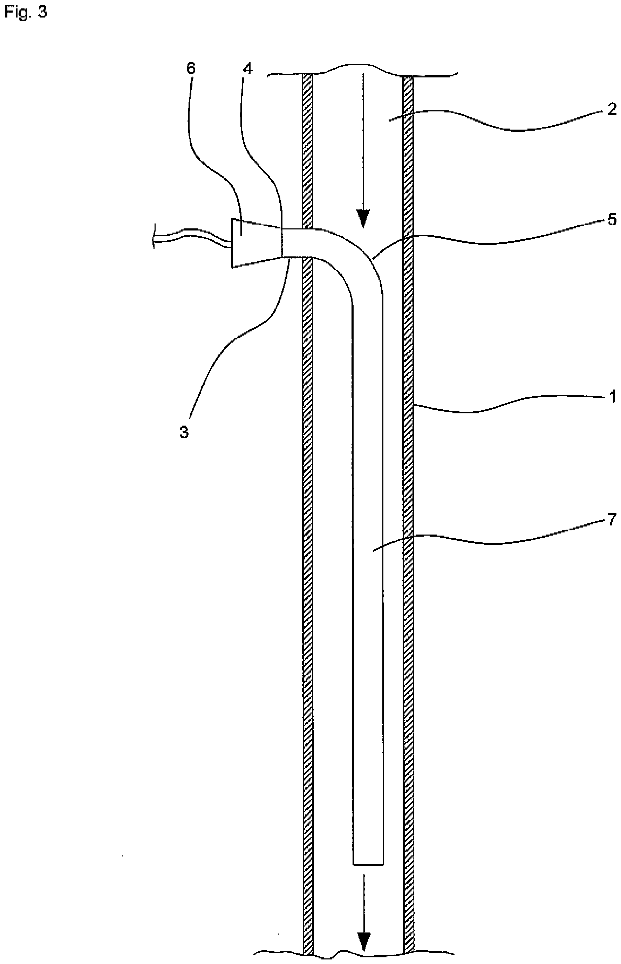

Fig. 3 zeigt ein weiteres Ausführungsbeispiel der erfindungsgemäßen Vorrichtung. In diesem Ausführungsbeispiel erfolgt die Behandlung des Fluids 2 ebenfalls in einem Durchströmverfahren, wobei das Behältnis 1 vorliegend als Rohrbeziehungsweise Rohrabschnitt mit einem geringen Durchmesser ausgebildet ist.

Das UV-Leiterelement 3 weist vorliegend nur eine einzelne, flexible Faser 7 auf und ist ebenfalls so mit dem Behältnis 1 verbunden, dass der Abstrahlabschnitt 5 im Inneren des Behältnisses 1 angeordnet ist.

Der besondere Vorteil der Vorrichtung im hier dargestellten Ausführungsbeispiel besteht darin, dass aufgrund des vergleichsweise geringen Durchmessers der Faser 7, der für diese notwendige Raum innerhalb des Behältnisses 1 entsprechend klein gehalten werden kann. Auf diese Weise wird es insbesondere ermöglicht, dass die Vorrichtung in Behältnissen mit sehr geringen Innendurchmessern, wie es beispielsweise bei Trinkwasserrohren oder -leitungen der Fall ist, angeordnet werden kann.

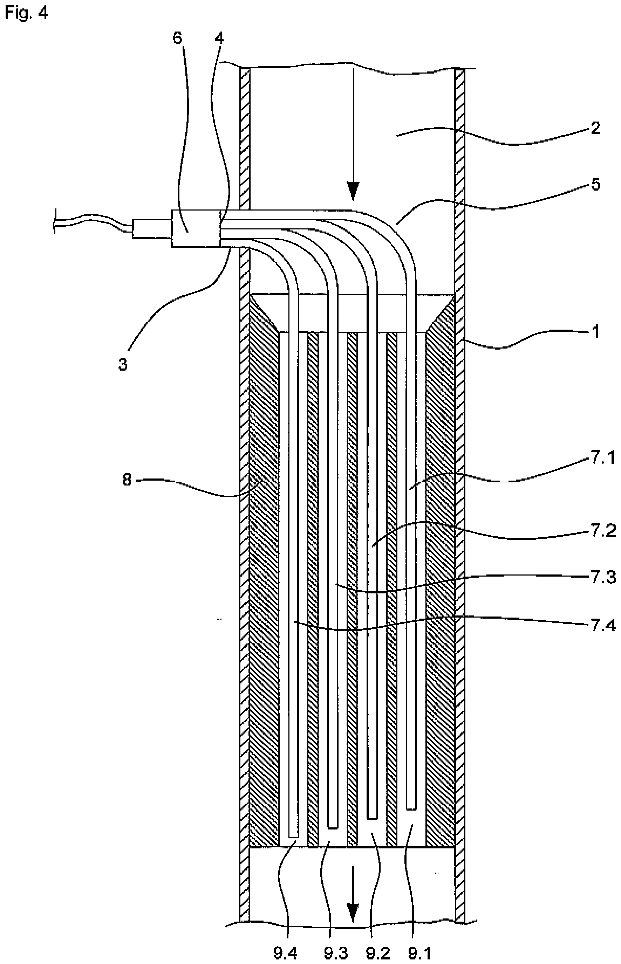

Die Fig. 4 zeigt ein weiteres Ausführungsbeispiel der erfindungsgemäßen Vorrichtung. Auch in diesem Fall erfolgt die Behandlung des Fluids 2 in einem Durchströmverfahren, wobei die Vorrichtung vorliegend zusätzlich ein Durchströmungselement 8 aufweist.

Das Durchströmungselement 8 verfügt über mehrere Durchströmungsausnehmungen 9, welche zum einen der Aufnahme und Separation der Fasern 7 dienen und durch welche zum anderen das Fluid 2 während der Behandlung strömt. Wie aus der Fig. 4 ersichtlich unterliegt das Fluid 2 während des Durchströmens der Durchströmungsausnehmungen 9 dem unmittelbaren Wirkungsbereich jeder Faser 7.

### Verwendete Bezugszeichen

- 1: Behältnis
- 2: Fluid
- 3: UV-Leiterelement
- 4: Einleitungsabschnitt
- 5: Abstrahlabschnitt
- 6: UV-Strahlungsquelle
- 7: Fasern
- 8: Durchströmungselement
- 9: Durchströmungsausnehmungen

## Patentansprüche

1. Vorrichtung zur Behandlung eines Fluids mit UV-Strahlung,
aufweisend ein Behältnis (1), in welchem zu behandelndes Fluid (2) aufnehmbar ist, und aufweisend ein strangförmiges UV-Leiterelement (3) mit einem Einleitungsabschnitt (4) und einem Abstrahlabschnitt (5), wobei das UV-Leiterelement (3) eine Mehrzahl Fasern (7) aufweist, wobei der Abstrahlabschnitt (5) im Inneren des Behältnisses (1) angeordnet ist und wobei in dem Einleitungsabschnitt (4) die UV-Strahlung einleitbar ist und wobei in dem Abstrahlabschnitt (5) eine laterale Abstrahlung der UV-Strahlung in das zu behandelnde Fluid (2) bereitstellbar ist, sowie aufweisend eine UV-Strahlungsquelle (6), mit welcher die UV-Strahlung zur Einleitung in das UV-Leiterelement (3) bereitstellbar ist,
**dadurch gekennzeichnet,**
**dass** ein Durchströmungselement (8) innerhalb des Behältnisses (1) angeordnet ist, welches Durchströmungsausnehmungen (9) aufweist, welche von dem zu behandelnden Fluid (2) durchströmbar sind und wobei die Fasern (7) in den Durchströmungsabschnitten angeordnet sind.

2. Vorrichtung nach Anspruch 1,
wobei die UV-Strahlungsquelle (6) und der Einleitungsabschnitt (4) des UV-Leiterelements (3) außerhalb des Behältnisses (1) angeordnet sind.

3. Vorrichtung nach einem der vorhergehenden Ansprüche,
wobei das UV-Leiterelement (3) aus Silikatglas, insbesondere aus Kieselglas besteht.

4. Vorrichtung nach einem der vorhergehenden Ansprüche,
wobei die Durchströmungsausnehmungen (9) eine längliche Erstreckung aufweisen und wobei die Fasern (7) im Wesentlichen achsparallel zu der länglichen Erstreckung in den Durchströmungsausnehmungen (9) angeordnet sind.

## Claims

1. A device for treating a fluid with UV radiation,
comprising a container (1), in which fluid (2) to be treated can be received, and comprising a strand-shaped UV conductor element (3) with an input section (4) and an emission section (5), wherein the UV conductor element (3) has a plurality of fibres (7), wherein the emission section (5) is arranged in the interior of the container (1), and wherein the UV radiation can be introduced into the input section (4), and wherein a lateral emission of the UV radiation into the fluid (2) to be treated can be provided in the emission section (5), and, furthermore, comprising a UV radiation source (6) by means of which the UV radiation can be provided for introduction into the UV conductor element (3),
**characterized in**
**that** a flow-through member (8) is arranged inside the container (1) which has flow-through recesses (9) through which the fluid (2) to be treated can flow, and wherein the fibres (7) are arranged in the flow-through sections.

2. The device according to claim 1,
wherein the UV radiation source (6) and the input section (4) of the UV conductor element (3) are arranged outside the container (1).

3. The device according to one of the preceding claims,
wherein the UV conductor element (3) is made of silicate glass, in particular silica glass.

4. The device according to one of the preceding claims,
wherein the flow-through recesses (9) have an elongate extension, and wherein the fibres (7) are provided substantially in an axially parallel arrangement to the elongate extension in the flow-through recesses (9).

## Revendications

1. Dispositif de traitement d'un fluide par rayonnement UV,
comprenant un contenant (1) pouvant accueillir le fluide à traiter (2), un élément conducteur d'UV en forme de filament (3) présentant un segment d'introduction (4) et un segment d'émission (5), où l'élément conducteur d'UV présente une pluralité de fibres, le segment d'émission (5) étant disposé à l'intérieur du contenant (1) et le rayonnement UV pouvant être introduit dans le segment d'introduction (4) et une émission latérale du rayonnement UV dans le fluide à traiter (2) pouvant se produire dans le segment d'émission (5), ainsi que comprenant une source de rayonnement UV (6) qui peut produire le rayonnement UV destiné à être introduit dans l'élément conducteur d'UV (3)
est **caractérisée en ce**
**qu'**un élément de circulation (8) est disposé à l'intérieur du contenant (1), lequel présente des évidements de circulation (9) qui peuvent être traversés par le fluide à traiter (2) et les fibres (7) sont disposées dans les sections de circulation écoulement.

2. Dispositif suivant la revendication 1,
où la source de rayonnement UV (6) et le segment d'introduction (4) de l'élément conducteur d'UV (3) sont disposés en dehors du contenant (1).

3. Dispositif suivant l'une quelconque des revendications précédentes où l'élément conducteur d'UV (3) est constitué de verre de silicate, en particulier de verre de silice.

4. Dispositif suivant l'une quelconque des revendications précédentes
où les évidements de circulation (9) ont une forme allongée et les fibres (7) sont disposées sensiblement parallèlement à l'axe de l'extension longitudinale dans les évidements de circulation (9).
